# FASCICULE DE BREVET EUROPEEN

(11) **EP 3 856 733 B1**
(45) Date de publication et mention de la délivrance du brevet: **29.12.2021**
(21) Numéro de dépôt: 20812121.0
(22) Date de dépôt: 01.12.2020
(51) Int. Cl.: C07D 317/20

(54) **PROCÉDÉ DE FABRICATION DU (2,2-DIMÉTHYL-1,3-DIOXOLAN-4-YL)MÉTHANOL**
VERFAHREN ZUR HERSTELLUNG VON (2,2-DIMETHYL-1,3-DIOXOLAN-4-YL)METHANOL
METHOD FOR MANUFACTURING (2,2-DIMETHYL-1,3-DIOXOLAN-4-YL)METHANOL

(30) Priorité: 03.12.2019 FR 1913682
(43) Date de publication de la demande: 04.08.2021
(73) Titulaire: Deasyl SA, 1228 Plan-les-Ouates (CH); Ecole Nationale Superieure de Chimie de Paris, 75005 Paris (FR); Centre National de la Recherche Scientifique (CNRS), 75016 Paris (FR)
(72) Inventeur: LEN, Christophe, 60150 VILLERS SUR COUDUN (FR); KHODADADI, Mohamad, 75014 PARIS (FR); THIEL, Julien, 74930 ARBUSIGNY (FR); LACOSTE, François, 92200 NEUILLY-SUR-SEINE (FR)
(74) Mandataire: Jacobacci Coralis Harle
(86) Numéro de dépôt international: PCT/EP2020/084143
(87) Numéro de publication internationale: WO 2021/110688

(56) Documents cités:
- AMIN TALEBIAN-KIAKALAIEH ET AL: "A Review on the Catalytic Acetalization of Bio-renewable Glycerol to Fuel Additives", FRONTIERS IN CHEMISTRY, vol. 6, 573, 26 novembre 2018 (2018-11-26) , XP055716930, DOI: 10.3389/fchem.2018.00573 cité dans la demande
- SANDRO GUIDI ET AL: "Towards a Rational Design of a Continuous-Flow Method for the Acetalization of Crude Glycerol: Scope and Limitations of Commercial Amberlyst 36 and AlF3.3H2O as Model Catalysts", MOLECULES, vol. 21, no. 5, 657, 18 mai 2016 (2016-05-18), XP055716810, ISSN: 1433-1373, DOI: 10.3390/molecules21050657
- JAY S. CLARKSON ET AL: "Continuous Reactor Technology for Ketal Formation: An Improved Synthesis of Solketal", ORGANIC PROCESS RESEARCH & DEVELOPMENT, vol. 5, no. 6, 16 novembre 2001 (2001-11-16), pages 630-635, XP002580240, ISSN: 1083-6160, DOI: 10.1021/OP000135P [extrait le 2001-07-21]
- T. REICHSTEIN ET AL: "Überführung der Salze von acetonierten Zuckersäuren in ihre Methylester", HELVETICA CHIMICA ACTA, vol. 18, no. 1, 1935, pages 598-601, XP055716783, DOI: 10.1002/hlca.19350180178

## Description

### Domaine technique de l'invention

La présente invention se rapporte à un procédé de fabrication du (2,2-Diméthyl-1,3-dioxolan-4-yl) méthanol, encore appelé solkétal.

En particulier, la présente invention concerne un procédé de fabrication de solkétal, effectué par microbroyage à une certaine température, d'un mélange de départ comprenant au moins du glycérol, de l'acétone et un catalyseur particulier.

### Etat de la technique

Le solkétal (2,2-Diméthyl-1,3-dioxolan-4-yl)méthanol, encore appelé le 1,2-O-isopropylidene-glycérol, N°CAS 100-79-8) est une molécule comprenant un groupement isopropylidène et un groupement hydroxyméthyle correspondant à la formule Chem.1 ci-dessous.

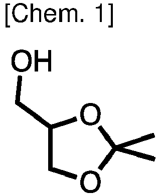

Le solkétal est un composé utile dans de nombreux domaines, par exemple dans le domaine pharmaceutique comme intermédiaire de synthèse, dans le domaine des polymères comme solvant et plastifiant (Maksimov et al., « Preparation of High Octane Oxygenate Fuel Components from Plant Derived Polyols » Pet. Chem. 51, 61-69, (2011)). Le solkétal a également montré des propriétés intéressantes comme additive de fuel en augmentant l'indice d'octane et en réduisant la formation de gomme (Mota et al., « Glycerin derivatives as fuel additives: the addition of glycerol/acetoneketal (solketal) in gasolines », Energy Fuels 24, 2733-2736 (2010); Silva et al., « Glycerolacetals as anti-freezing additives for biodiesel », BioresourTechnol. 101, 6225-6229 (2010) et Garcia et al., "New class of acetal derived from glycerin as a biodiesel fuel component", Energy Fuels 22, 4274-80 (2008)).

Généralement, le solkétal est préparé par acétalisation à partir du glycérol et de l'acétone ou du 2,2-diméthoxypropane en milieu acide (Chem.2 ci-dessous).

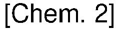

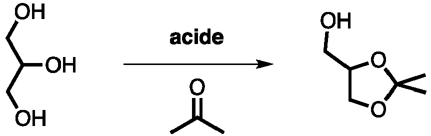

Le catalyseur acide utilisé pour réaliser la réaction de synthèse est généralement un catalyseur homogène comme H₂SO₄, HCl, mais d'autres acides peuvent être utilisés, tels que des acides de Lewis ou des catalyseurs acides hétérogènes (Talebian-Kiakalaiehet al., « A review on the catalytic acetalization of bio-renewableglycerol to fuel additives », Front. Chem. 6, 573, (2018)). Le plus souvent la réaction est réalisée dans un réacteur discontinu, bien que quelques exemples montrent la possibilité d'utiliser un réacteur continu.

Différents procédés de fabrication de solkétal ont été proposés dans l'art antérieur.

La publication de Torres et al., « Glycerolketais : synthesis and profits in biodiesel blends » Fuel 94, 614-616, (2012) décrit en particulier de faire réagir du glycérol (10,9 mmol) dans de l'acétone en excès (106 mmol) en présence d'acide sulfurique (0,10 mmol) comme catalyseur homogène acide à 35 °C pendant 1 heure. Après évaporation de l'acétone, une solution saturée de NaHCO₃ et de l'acétate d'éthyle sont ajoutés et la phase organique est évaporée pour conduire au solkétal avec un rendement de 80%. L'utilisation d'acide sulfurique comme catalyseur nécessite cependant une étape de neutralisation et d'extraction. Or, l'étape d'extraction mène à une perte notable de solkétal dans la phase aqueuse (de l'ordre de 20%).

La publication de Nanda et al., "Thermodynamic and kinetic studies of a catalytic process to convert glycerol into solketal as an oxygenated fuel additive", Fuel 117, 470-477, (2014), décrit de faire réagir du glycérol (197 mmol) et l'acétone en excès (394 mmol) en présence d'Amberlyst (1% massique) comme catalyseur hétérogène acide dans l'éthanol (197 mmol) à 25°C pendant 3,7 heures. Dans ces conditions, le solkétal est obtenu avec un rendement de 74%. L'utilisation d'éthanol en quantité équimolaire permet de solubiliser le glycérol et l'acétone.

La publication de Da Silva et al.,"Solvent-free heteropolyacid-catalyzed glycerol ketalization at room temperature", RSC. Adv. 5, 44499-44506, (2015), décrit de faire réagir le glycérol (9,23 mmol) en présence d'hétéroacide H₃PW₁₂O₄₀ (3% molaire en H⁺) comme catalyseur homogène acide dans de l'acétone (185 mmol) comme solvant à 25°C pendant 2 heures. Cela conduit au solkétal avec un rendement de 82% (conversion 83%, sélectivité 98%).

Le document WO 2009/141702 décrit un procédé de fabrication de solkétal qui est obtenu en flux continu par réaction entre du glycérol et de l'acétone à une température allant de 50 à 150°C. Ce dernier est ajouté en faible quantité (5 à 20 mol%) à chaque cycle à 60°C. Après plusieurs heures de flux continu (de 2 à 8 heures, généralement de 3 à 6 heures), le solkétal est obtenu.

Lors de cette réaction, l'acétone est d'abord protonée ce qui augmente son électrophilie. Le doublet non-liant de l'hydroxyle primaire du glycérol nucléophile attaque l'atome de carbone de l'acétone activé. Après déprotonation de l'hydroxyle primaire du glycérol et protonation de l'hydroxyle quaternaire, le doublet non-liant de l'hydroxyle secondaire du glycérol nucléophile attaque l'atome de carbone libérant une molécule d'eau. La dernière étape est la déprotonation libérant le catalyseur acide (voir Chem.3 ci-dessous).

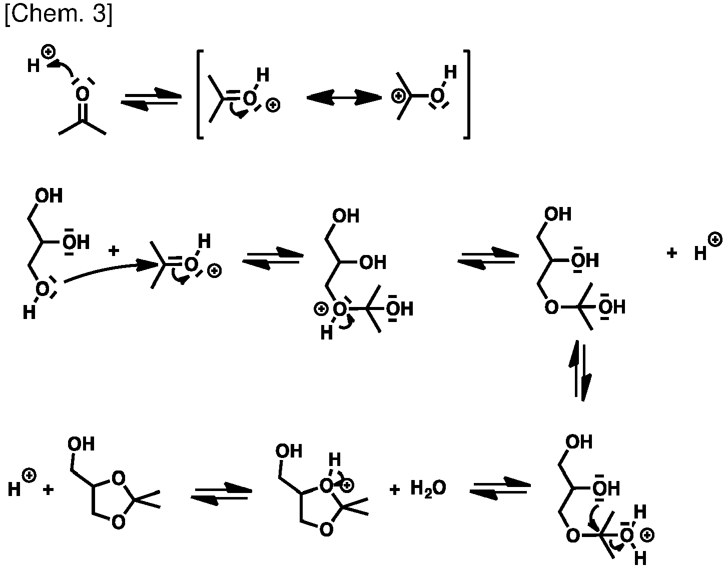

Toutefois, la réaction d'acétalisation du glycérol en solkétal présente certains inconvénients puisqu'elle nécessite en général l'utilisation d'acide homogène ou hétérogène en quantité catalytique importante (quantité en masse supérieure à 0,004% par rapport à la masse totale des composés de départ) et un temps de réaction long supérieur à 1 heure. L'obstacle majeur de la production de solkétal est la constante d'équilibre faible. Pour pallier ce problème, l'utilisation d'un ratio acétone-glycérol important ou le piégeage de l'eau formée permet d'augmenter la productivité en solkétal en déplaçant l'équilibre de la réaction.

Ainsi, ces procédés de l'art antérieur souffrent du fait qu'ils présentent soit un rendement insatisfaisant, soit qu'ils nécessitent une température élevée et/ou un temps de réaction très long (pouvant aller par exemple de 2 à 8 heures).

La publication de Amin Talebian-Kiakalaieh et al.,"A review on the catalytic acetalization of bio-renewable glycerol to fuel additives", frontiers in chemistry, November 2018, répertorie les nombreuses méthodes d'acétalisation catalytique du glycérol, et en particulier la réaction avec de l'acétone afin de préparer du solkétal.

Il existe donc un besoin dans l'état de la technique pour de nouveaux procédés de fabrication de solkétal, de préférence industriellement exploitable, et qui soient alternatifs ou améliorés par rapport aux procédés connus.

La présente invention a donc pour objet de fournir un procédé de fabrication de solkétal, qui évite au moins en partie les inconvénients susmentionnés. En particulier, la présente invention a pour but de fournir un nouveau procédé de fabrication de solkétal qui soit industriellement exploitable, tout en ne nécessitant pas un chauffage trop important et/ou un temps de réaction trop long.

### Présentation de l'invention

A cet effet, la présente invention propose un procédé de fabrication du (2,2-diméthyl-1,3-dioxolan-4-yl)méthanol (nommé ci-après solkétal) qui comprend au moins les étapes suivantes :
(1) le broyage des réactifs suivants, dits réactifs de départ, comprenant au moins : du glycérol, un catalyseur choisi parmi un acide de Lewis dur comprenant au moins un métal de transition, et de l'acétone, le ratio molaire (glycérol) : (acétone) étant inférieur ou égal à 0,8, de préférence inférieur ou égal à 0,7, à une température ambiante supérieure ou égale à 50°C, de préférence supérieure ou égale à 56°C, dans un broyeur tridimensionnel à microbilles pendant un temps de séjour inférieur ou égal à 15 minutes, de préférence inférieur ou égal à 10 minutes et en particulier inférieur ou égal à 5 minutes;
(2) la récupération en sortie du broyeur d'une composition finale comprenant du solkétal et le cas échéant, un ou des sous-produits correspondant au(x) réactif(s) de départ n'ayant pas réagi(s) et/ou au 1,3-*O*-isopropylidene-glycérol, et
(3) optionnellement, la séparation du solkétal du ou desdits sous-produits.

Les inventeurs ont mis au point un procédé qui, de manière surprenante et inattendue, permet la synthèse de solkétal à une température relativement basse (aux alentours de 50°C-70°C), en une seule étape de broyage, et ce en des temps très court (le temps de séjour des réactifs dans le broyeur est inférieur à 15 minutes et est généralement inférieur à 5 minutes versus 2 à 8 heures pour le procédé selon le document WO 2009/141702).

Comme cela sera démontré dans les essais expérimentaux ci-dessous, le procédé de l'invention utilisant un broyeur particulier, à savoir un broyeur tridimensionnel à microbilles, combiné à des conditions de réaction particulières (choix et concentration molaire des réactifs de départ, température de réaction, débit de passage, etc.) permet de fabriquer du solkétal avec un rendement généralement supérieur ou égal à 80% et en particulier supérieur ou égal à 99%.

Le procédé selon l'invention présente également les avantages d'avoir un prix de revient très réduit (les matières premières utilisées sont en effet largement disponibles, non polluantes et peu coûteuses) et de présenter une excellente reproductibilité, ce qui le démarque davantage des procédés décrits dans les arts antérieurs. Le procédé selon l'invention présente également l'avantage de pouvoir être mis en œuvre de façon continue. Or, ces caractéristiques sont importantes pour une application à l'échelle industrielle.

En outre, malgré les nombreuses recherches conduites sur la synthèse du solkétal, aucune n'a suggéré le procédé susmentionné et en particulier une étape de broyage dans un broyeur tridimensionnel à microbilles à partir des réactifs de départ.

D'autres caractéristiques non limitatives et avantageuses du procédé de fabrication de solkétal conforme à l'invention, prises individuellement ou selon toutes les combinaisons techniquement possibles, sont les suivantes :
- le procédé comprend une étape préalable (0) dans laquelle les réactifs de départ dont au moins le glycérol et l'acétone, voire également de préférence ledit catalyseur, sont préalablement mélangés pour former une composition de départ ;
- lors de l'étape préalable (0), la composition de départ est préalablement chauffée à une température supérieure ou égale à 50°C, de préférence supérieure ou égale à 56°C, de sorte que la température lors de l'étape de broyage (1) soit supérieure ou égale à 50°C, de préférence supérieure ou égale à 52°C et de manière encore plus préférée aux alentours de 56°C ;
- lors de l'étape de broyage (1), les réactifs de départ sont chauffés au sein du broyeur tridimensionnel à microbilles qui comprend un dispositif de chauffage, de préférence un dispositif de chauffage par induction ;
- la pression lors de l'étape de broyage (1) va de 0,05 à 20 MPa, de préférence de 0,08 à 0,5 MPa et est typiquement de l'ordre de 0,1 MPa ;
- le catalyseur acide de Lewis dur comprenant au moins un métal de transition est choisi parmi FeCl₃, AlCl₃, CrCl₃, MnSO₄ ou un de leurs mélanges ;
- le glycérol est anhydre ou présente une teneur en eau, en masse, par rapport à la masse totale du glycérol allant de 0 à 10%, de préférence de 0 à 5% ;
- les microbilles sont de forme sphérique et présentent un diamètre moyen allant de 0,05 mm à 4 mm, de préférence de 0,2 à 3 mm, en particulier de 0,3 à 2 mm et typiquement de l'ordre de 0,5 à 1 mm ;
- les microbilles présentent une dureté de Vickers mesurée selon la norme EN ISO 6507-1 supérieure ou égale à 900 HV1, de préférence allant de 900 HV1 à 1600 HV1, typiquement allant de 1000 à 1400 HV1 ;
- les microbilles présentent une masse volumique réelle allant de 2 à 15 g/cm³ ;
- l'étape de broyage (1) s'effectue à une température ambiante allant de 50°C à 70°C, en particulier de 55°C à 60°C et en général est de l'ordre de 56°C ;
- dans lequel le broyeur tridimensionnel à microbilles comprend au moins :
   une chambre de broyage stationnaire de forme générale cylindrique s'étendant selon un axe longitudinal XX, ladite chambre étant remplie au moins, en partie, par lesdites microbilles et comprend : à une première extrémité au moins une entrée servant à introduire lesdits réactifs de départ, et à une seconde extrémité, une sortie comportant un moyen de séparation apte à n'évacuer que ladite composition finale ;
   un agitateur, disposé dans la chambre de broyage stationnaire, se présentant sous forme d'une tige allongée selon l'axe longitudinal XX, ledit agitateur étant apte à mettre en mouvement l'ensemble microbilles/ lesdits réactifs de départ ;
- dans lequel les microbilles représentent, en volume, par rapport au volume total de la chambre stationnaire de 50% à 85%, de préférence de 55% à 70% ;
- dans lequel le broyeur fonctionne en continu.

Pour le reste de la description, à moins qu'il n'en soit spécifié autrement, l'indication d'un intervalle de valeurs « de X à Y » ou « entre X et Y », dans la présente invention, s'entend comme incluant les valeurs X et Y.

Selon l'invention, « un ou des sous-produits » présent dans la composition finale correspond(ent) au(x) réactif(s) de départ n'ayant pas réagi(s) comme par exemple le catalyseur ou encore le glycérol (notamment si le rendement de la réaction est inférieur ou égal à 99%) et le cas échéant, à un coproduit pouvant se former lors de la réaction qui est le 1,3-*O*-isopropylidene-glycérol.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

L'invention sera mieux comprise et d'autres buts, détails, caractéristiques et avantages de celle-ci apparaîtront plus clairement à la lecture de la description suivante d'exemples de réalisation, en référence aux figures annexées dans lesquelles :
[Fig. 1] représente une vue en coupe, selon un plan de coupe passant par l'axe longitudinal XX, d'un broyeur tridimensionnel selon un premier mode de réalisation de l'invention comprenant notamment un dispositif de chauffage par induction ;
[Fig. 2] représente, selon des plans de coupe passant par l'axe longitudinal XX et par l'axe AA, différentes variantes de réalisation de broyeurs tridimensionnels selon l'invention comportant chacun un dispositif de chauffage et au moins un agitateur supportant éventuellement un autre organe de mélange :(a) l'agitateur comprend plusieurs autres organes de mélange conformément au broyeur de la figure 1, (b) l'agitateur comporte en plus des doigts aptes à coopérer avec les autres organes de mélange et (c) l'agitateur ne comporte pas d'organes de mélange et de doigts ;
[Fig. 3] représente un chromatogramme correspondant à la synthèse du solkétal à partir du glycérol après un temps de séjour de 1 minutes (temps de réaction au sein du broyeur tridimensionnel) ; et
[Fig. 4] représente un chromatogramme correspondant à la synthèse du solkétal à partir du glycérol après un temps de séjour de 2,37 minutes (temps de réaction au sein du broyeur tridimensionnel).

Il est à noter que, sur ces figures, les éléments structurels et/ou fonctionnels communs aux différentes variantes peuvent présenter les mêmes références.

Les inventeurs se sont attachés au développement d'un nouveau procédé de fabrication de solkétal adapté pour être mis en œuvre à une échelle industrielle et ce en un temps très court.

Ainsi, la présente invention concerne un procédé de fabrication du (2,2-diméthyl-1,3-dioxolan-4-yl)méthanol (nommé ci-après solkétal) qui comprend au moins les étapes suivantes :
(1) le broyage des réactifs suivants, dits réactifs de départ, comprenant au moins : du glycérol et un catalyseur choisi parmi un acide de Lewis dur comprenant au moins un métal de transition (appelé ci-après « le catalyseur »), avec de l'acétone, le ratio molaire (glycérol) : (acétone) étant inférieur ou égal à 0,8 ; de préférence inférieur ou égal à 0,7, à une température ambiante supérieure ou égale à 50°C, de préférence supérieure ou égale à 56°C, dans un broyeur tridimensionnel à microbilles pendant un temps de séjour inférieur ou égal à 15 minutes, de préférence inférieur ou égal à 10 minutes et en particulier inférieur ou égal à 5 minutes;
(2) la récupération en sortie du broyeur d'une composition finale comprenant du solkétal et le cas échéant, un ou des sous-produits correspondant au(x) réactif(s) de départ n'ayant pas réagi(s) et/ou au 1,3-O-isopropylidene-glycérol, et
(3) optionnellement, la séparation du solkétal du ou desdits sous-produits.

Généralement, le procédé comprend une étape préalable (0) dans laquelle les réactifs de départ dont au moins le glycérol et l'acétone et de préférence le catalyseur sont préalablement mélangés pour former une composition de départ.

En particulier, le procédé selon l'invention permet de réaliser la synthèse suivante (Chem 4):

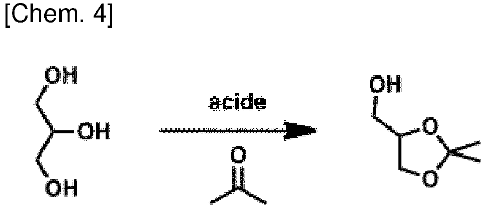

Le composé « acide » représentant ici le catalyseur selon l'invention, à savoir le catalyseur acide de Lewis dur comprenant au moins un métal de transition.

Par « acide de Lewis dur » selon l'invention, on entend un acide de Lewis dont le centre accepteur d'électron est peu polarisable. Le critère de dureté d'un acide est notamment tel que défini dans le principe Hard and Soft Acid and Base (ci-après HSAB) connu de l'homme du métier. Les acides durs sont par exemple le fer (III), le Chrome (IV), l'aluminium (III), le manganèse (II), etc. Le fer (II) et le cuivre (II) sont de façon connus des acides métalliques intermédiaires et le cuivre (I) est un acide mou.

Par conséquent, selon le procédé de l'invention et contrairement aux procédés de l'art antérieur tel que le procédé du document WO 2009/141702, l'équilibre de la réaction est déplacé vers la formation du solkétal ; il n'y a pas besoin de piéger l'eau ou d'utiliser un ratio acétone-glycérol important permettant de déplacer la constante d'équilibre de la réaction et ainsi d'augmenter la productivité en solkétal.

Eventuellement, si la réaction n'est pas totale et que le rendement en solkétal est supérieur ou égal à 80% (cela dépend notamment des paramètres de réaction, tels que les proportions molaires des réactifs de départ par exemple en acétone et/ou de la température lors de l'étape broyage), du 1,3-O-isoprolylidene-glycérol peut également se former en tant que sous-produit (Chem 5) :

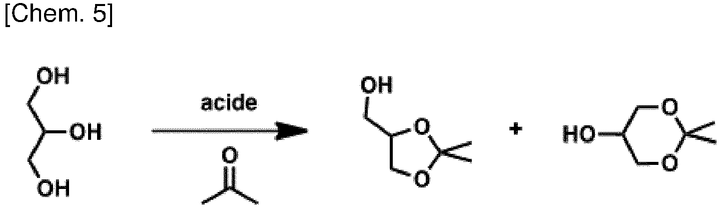

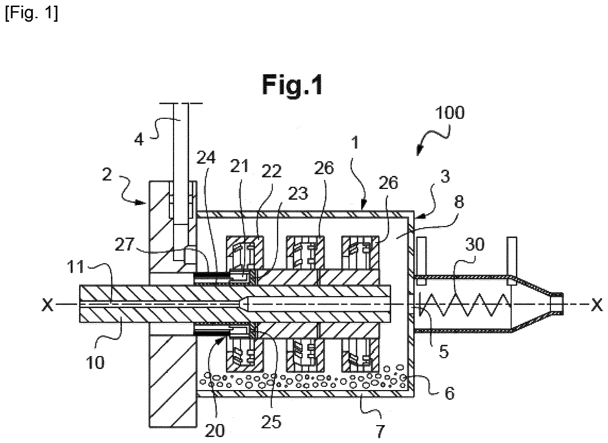

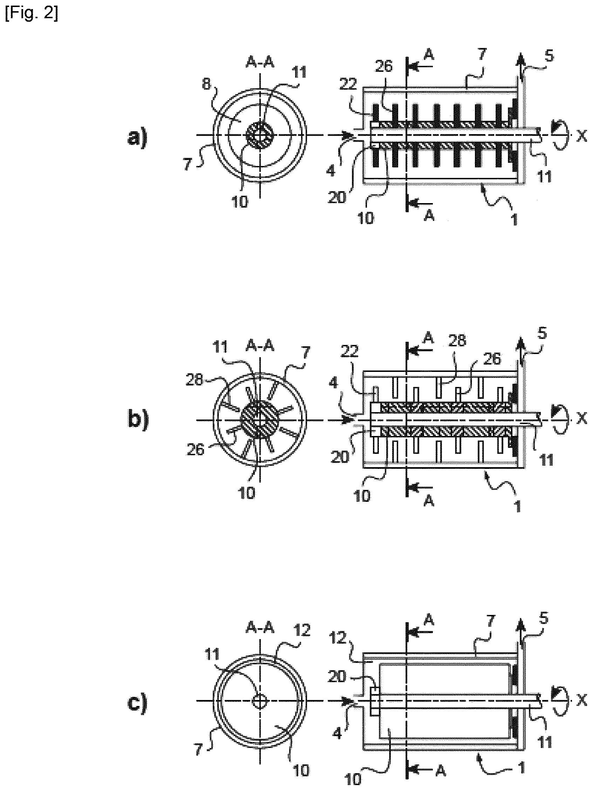

Pour mieux faire comprendre le procédé objet de l'invention, un broyeur tridimensionnel à microbilles susceptible de permettre la synthèse du solkétal, et faisant ainsi partie de l'invention, va tout d'abord être décrit ci-dessous en se référant aux figures 1 et 2.

Tel qu'illustré sur les figures 1 et 2, le broyeur tridimensionnel 100 comprend au moins une chambre de broyage stationnaire 1 présentant une paroi 7 de forme générale cylindrique qui enveloppe un intérieur 8.

La paroi 7 s'étend selon un axe longitudinal XX, avantageusement horizontal.

Cette chambre de broyage stationnaire 1 est configurée pour recevoir et mélanger au moins les réactifs de départs, à savoir le glycérol, l'acétone et le catalyseur ou le cas échéant, la composition de départ (mode de réalisation particulier où les réactifs de départ sont préalablement mélangés).

Cette chambre de broyage stationnaire 1 est remplie partiellement avec au moins des corps de broyage 6, tels que des microbilles 6 qui vont permettre le broyage et le mélange des réactifs d'une manière intense et efficace.

La chambre stationnaire 1 comprend, à une première extrémité 2 (amont), une entrée 4 qui débouche au sein de la chambre de broyage stationnaire 1 et qui sert à introduire le ou les réactifs de départ ou la composition de départ.

Cette entrée 4 peut également servir à introduire les microbilles 6 avant la mise en œuvre du broyeur 100. Comme on le verra ci-après, la dimension et la nature des microbilles 6 peuvent légèrement variées.

La chambre de broyage 100 comprend, à une seconde extrémité 3 (aval), une sortie 5 qui conduit vers l'extérieur et qui est configurée pour évacuer la composition finale formée dans la chambre de broyage stationnaire 1.

La sortie 5 comporte en général un moyen de séparation (non représenté), tel qu'un tamis ou une grille, adapté à évacuer seulement la composition finale et à retenir par conséquent les microbilles 6 lorsque le broyeur 100 est en fonctionnement.

En particulier, l'entrée 4 est généralement reliée à au moins une pompe, par exemple péristaltique (non représentée). Ce ou ces pompe(s) permet(tent) d'amener le ou les réactifs de départ ou encore la composition de départ préalablement préparée, à l'intérieur de la chambre de broyage stationnaire 1.

Les réactifs de départ ou la composition de départ peuvent, par exemple, être contenus dans un récipient, tel qu'une cuve. La pompe permet en plus, lors du fonctionnement du broyeur tridimensionnel 100, d'amener les réactifs de départ ou la composition de départ avec un certain débit qui est réglable, appelé par la suite « débit de passage ». Ce débit de passage forme en outre un courant dans la chambre stationnaire 1 permettant d'entraîner ces réactifs de départ et/ou cette composition de départ de l'entrée 4 vers la sortie 5.

Le broyeur tridimensionnel 100 comprend également un agitateur 10 qui comporte une tige allongée 11 selon l'axe longitudinal XX et qui s'étend principalement aux alentours de la première extrémité 2 à au-delà de la seconde extrémité 3 de la chambre stationnaire 1.

Cette tige allongée 11 s'étend avantageusement coaxialement à l'axe longitudinal XX précité.

Cet agitateur 10 est notamment apte à pivoter de sorte à mettre en mouvement, en plus du débit de passage susmentionné, l'ensemble corps de broyage 6 et réactifs de départ/composition de départ.

En particulier, l'agitateur 10 est configuré pour tourner sur lui-même, selon l'axe longitudinal XX, via une tige allongée 11 (ou arbre rotatif), pour impartir au sein de la chambre stationnaire 1 un mouvement tourbillonnaire au mélange initial et effectuer ainsi un brassage intense entre ce mélange initial et les microbilles 6 présentes dans la chambre 1 le long de la surface interne de la paroi 7 de cette chambre 1.

En particulier, l'agitateur 10 via sa tige allongée 11 peut présenter une vitesse de rotation supérieure ou égale à 100 tours par minute, avantageusement supérieure ou égale à 1000 tours par minute (tr/min), de préférence supérieure ou égale à 2000 tours par minute et typiquement supérieure ou égale à 2500 tours par minute.

Au sens de l'invention, « une vitesse de rotation supérieure ou égale à 100 » comprend les valeurs suivantes : 100 ; 150 ; 200 ; 250 ; 300 ; 350 ; 400 ; 450 ; 500 ; 550 ; 600 ; 650 ; 700 ; 750 ; 800 ; 850 ; 900 ; 950 ; 1000 ; 1100 ; 1200 ; 1300 ; 1400 ; 1500 ; 1600 ; 1700 ; 1800 ; 1900 ; 2000 ; 2100 ; 2200 ; 2300 ; 2400 ; 2500 ; 2600 ; 2700 ; 2800 ; 2900 ; 3000 ; 3100 ; 3200 ; 3300 ; 3400 ; 3500 ; 3600 ; 3700 ; 3800 ; 3900 ; 4000, 4500 ; 5000 ; 5500 ; 6000 ; etc., ou tous intervalles compris entre ces valeurs.

En particulier, la vitesse de rotation de l'agitateur 10 est supérieure ou égale à 1500 tr/min, avantageusement supérieure ou égale à 1600 tr/min, en particulier supérieure ou égale à 1800 tr/min et typiquement supérieure ou égale à 2400 tr/min.

En général, l'agitateur 10 présente une vitesse de rotation allant par exemple de 1500 tr/min à 5000 tr/min, en particulier de 1550 tr/min à 4500 tr/min, de préférence de 1600 tr/min à 4000 tr/min et typiquement de 2400 à 3200 tr/min.

De préférence, la vitesse périphérique de l'agitateur est supérieure ou égale à 6 m/s, en particulier supérieure ou égale à 8 m/s.

Selon l'invention, une vitesse périphérique de l'agitateur supérieure ou égale à 6 m/s comprend les valeurs suivantes ou tout intervalle entre ces valeurs : 6 ; 7 ; 8 ; 9 ; 10 ; 11 ; 12 ; 13 ; 14; 15 ; 16 ; 17 ; 18 ; 19 ; 20, etc.

En général, la vitesse périphérique de l'agitateur va de 7 m/s à 20 m/s, de préférence de 8m/s à 16 m/s.

Par « vitesse périphérique de l'agitateur », on entend la vitesse de rotation multipliée par la circonférence du disque de l'agitateur.

La vitesse de rotation sera adaptée par l'homme du métier en fonction du broyeur tridimensionnel utilisé (broyeur de laboratoire ou broyeur industriel).

Par exemple, un broyeur tridimensionnel commercialisé par la société WAB (Willy A. Bachofen SARL) du type AP05 présente un agitateur ayant les caractéristiques suivantes : pour une fréquence de 80 Hz, une vitesse en tour par minute de 4800 et une vitesse périphérique en m/s de 16,0 ; tandis qu'un broyeur du type AP2, pour une fréquence de 70,8 Hz, présente une vitesse en tour par minute de 2730 et une vitesse périphérique en m/s de 16,0. Afin d'améliorer ce brassage, l'agitateur 10, tout comme la surface interne de la paroi interne 7 de la chambre 1, peuvent présenter diverses configurations possibles représentées par exemple sur la figure 2.

Selon une première configuration illustrée à la figure 2a, l'agitateur 10 comprend, le long de sa tige allongée 11, des organes de mélanges 22, 26 « rotatifs », disposés perpendiculairement à celle-ci.

Tel que cela sera décrit par la suite, un organe de mélange 22 (dit « premier organe de mélange ») peut correspondre également à un suscepteur du moyen de chauffage 20 selon l'invention et est ainsi différent des autres organes de mélanges 26 (dit « autres organes de mélange »).

Ce premier organe de mélange 22, ainsi que les autres organes de mélange 26, peuvent correspondre aux organes de mélange décrits dans le document US 5 597 126.

En particulier, ils peuvent comporter au moins deux disques circulaires parallèles l'un à l'autre, configurées pour mettre en mouvement les corps de broyages 6 (microbilles).

Le nombre de ces organes de mélange 22, 26 au sein de la chambre de broyage 1 peut varier de 2 à 8, de préférence de 2 à 5.

Ces organes de mélange 22, 26 permettent d'une part, d'améliorer le broyage des réactifs de départ et/ou de la composition de départ en brassant davantage les microbilles 6 et d'autre part, d'accélérer le temps de réaction.

Selon une deuxième configuration illustrée à la figure 2b, l'agitateur 10 peut également comprendre, le long de sa tige 11, un ou plusieurs organes de mélanges 22, 26 « rotatifs » et qui sont en outre aptes à coopérer avec des doigts 28 « fixes », disposés perpendiculairement par rapport à la paroi interne 7 de la chambre 1.

Un doigt 28 se présente notamment sous la forme d'un anneau qui s'étend perpendiculairement à partir de la paroi 7.

Pour cette configuration, les organes de mélange 22, 26 et les doigts 28 sont disposés en quinconce, à savoir les organes de mélange 22, 26 et les doigts 28 sont disposés de manière alternée dans la chambre 1.

Les doigts 28 forment ainsi des contres-doigts, disposés chacun entre deux organes de mélange 22, 26.

En outre, l'épaisseur de la tige 11 est augmentée par rapport à la configuration précédente (figure 2a) de sorte que la périphérie des organes de mélange 22, 26 soit proche de la paroi interne 7 et que celle des doigts 28 soit proche de la périphérie de la tige de l'agitateur 10.

Ainsi, dans cette configuration, le volume de la chambre est réduit par rapport à la configuration précédente, permettant par conséquent, un meilleur brassage entre les réactifs de départ et/ou la composition de départ, les microbilles 6 et la paroi interne 7 de la chambre 1.

Selon une troisième configuration le volume de la chambre 1 peut encore être réduit comme cela est illustré en figure 2c.

Selon ce mode, l'agitateur 10 présente un diamètre externe légèrement inférieur au diamètre interne de la chambre 1, formant ainsi une chambre annulaire 12 de faible volume disposée entre la paroi externe de l'agitateur 10 et la paroi interne 7 de la chambre 1. Les microbilles (non représentées) sont disposées dans cette chambre annulaire 12. Lors du fonctionnement de cette troisième configuration, les réactifs de départ et/ou la composition de départ sont/est introduite par l'entrée 4 avec un certain débit, qui va ensuite parcourir la chambre annulaire 12 jusqu'à la sortie 5, tout en étant brassée par les microbilles 6.

La géométrie de la chambre de broyage 1 et de l'agitateur 10 pourra être ajustée par l'homme du métier en fonction du rendement souhaité, ainsi que du temps de réaction désiré. Par exemple, il est également possible que la chambre de broyage 1 comprenne un accélérateur afin d'améliorer le broyage du mélange initial. Cet accélérateur étant connu de l'homme du métier, il ne sera pas détaillé ci-après.

En général, la chambre stationnaire présente un diamètre de 75 mm à 300 mm pour une longueur de 80 mm à 900 mm et un agitateur 10 présentant une taille allant de 65 mm à 260 mm. Ainsi, le volume de la chambre de broyage peut varier de 0,35 L à 600 L, de préférence de 0,35 L à 400 L, et typiquement de 0,35 L à 62 L.

Au sens de l'invention, « un volume de la chambre stationnaire 1 allant de 0,35 L à 600 L» comprend les valeurs suivantes : 0,35 ; 0,5 ; 0,8 ; 1 ; 2 ; 3 ; 4 ; 5 ; 6 ; 7 ; 8 ; 9 ; 10 ; 15 ; 20 ; 25 ; 30 ; 35 ; 40 ; 45 ; 50 ; 55 ; 60 ; 65 ; 70 ; 80 ; 85 ; 90 ;100 ; 110 ; 120 ; 130 ; 140 ; 150 ; 160 ; 170 ; 180 ; 190 ; 200 ; 210 ; 220 ; 230 ; 240 ; 250 ; 260 ; 270 ; 280 ; 290 ; 300 ; 350 ; 400 ; 450 ; 500 ; 550 ; 600, ou tous intervalles compris entre ces valeurs.

De préférence, les microbilles 6 logées dans la chambre de broyage 3 du broyeur 1 lors de son fonctionnement sont substantiellement de forme sphérique et présentent un diamètre moyen inférieur ou égal à 5 mm, généralement allant de 0,05 mm à 4 mm, de préférence de 0,2 à 3 mm, en particulier de 0,3 à 2 mm, et typiquement de l'ordre de 0,5 à 1 mm. De préférence, le diamètre des microbilles est inférieur ou égal à 1 mm et est typiquement de l'ordre de 0,05 mm à 1 mm.

Elles sont préférentiellement choisies parmi des microbilles présentant une dureté élevée et résistant relativement bien à l'abrasion.

En particulier, les microbilles 6 présentent une dureté de Vickers mesurée selon la norme EN ISO 6507-1 (2005) supérieure ou égale à 900 HV1, de préférence allant de 900 HV1 à 1600 HV1, typiquement allant de 1000 à 1400 HV1 et notamment allant de 110 à 1300 HV1.

Au sens de l'invention, « une dureté de Vickers supérieure ou égale à 900 HV1 » comprend les valeurs suivantes : 900 ; 910 ; 920 ; 930 ; 940 ; 950 ; 960 ; 970 ; 980 ; 990 ; 1000 ; 1010 ; 1020 ; 1030 ; 1040 ; 1050 ; 1060 ; 1070 ; 1080 ; 1090 ; 1000 ; 1110 ; 1120 ; 1130 ; 1140 ; 1150 ; 1160 ; 1170 ; 1180 ; 1190 ; 1200 ; 1300 ; 1400 ; 1500 ; 1600 ; 1700 ; etc., ou tous intervalles compris entre ces valeurs.

Avantageusement, elles présentent une masse volumique réelle élevée. En général, les microbilles selon l'invention ont une masse volumique réelle supérieure ou égale 2 g/cm³, en particulier allant de 2 à 15 g/cm³, de préférence de 3 à 12 g/cm³, et typiquement de 4 à 10 g/cm³.

Ainsi, les microbilles selon l'invention peuvent être des microbilles en céramique, (oxyde de zirconium ZrO₂, en silicate de zirconium ZrSiO₄) ; des microbilles en acier, des microbilles en carbure de tungstène, des microbilles en verre ou une de leurs combinaisons.

De préférence, les microbilles sont en céramiques car elles ne génèrent pas de pollution par leur usure.

En particulier, les microbilles sont en oxyde de zirconium.

Eventuellement, les microbilles en oxyde de zirconium peuvent être stabilisées par un autre oxyde, tel que l'oxyde de cérium, l'oxyde d'yttrium et/ou le silicium.

A titre d'exemples, les compositions suivantes, résumées dans le tableau 1 ci-dessous, conviennent pour former les microbilles selon l'invention :

**[Tableau 1]**

| **Composition des microbilles** | | **Dureté HV1** | **Masse volumique réelle (g/cm³)** | | **Fabricant** |
|---|---|---|---|---|---|
| Microbilles en oxyde de zirconium stabilisées par l'oxyde de cérium | | 1180 | ≥ 6,10 | Saint-Gobain (Zirmil^{®}YCeramicBeads) ou EIP (Procerox^{®} ZO Cer) | |
| | 80% de ZrO₂ | | | | |
| | 20% de CeO | | | | |
| Microbilles en oxyde de zirconium stabilisées par l'yttrium | | 1250 | ≥ 5,95 | EIP (Procerox^{®} ZO (Y)) | |
| | 95% ZrO₂ | | | | |
| | <5% Al₂O₃ | | | | |
| | Reste : Y₂O₃ | | | | |
| Microbilles en oxyde de zirconium stabilisées par l'yttrium et du silicium : | | > 700 | >4,80 | Saint-Gobain (ER120 CeramicBeads.) | |
| | 78% ZrO₂, | | | | |
| | 12% SiO₂, | | | | |
| | 5% Al₂O₃ et | | | | |
| | 4 % Y₂O₃ | | | | |
| Microbilles en silicate de zirconium ZrSiO₄ | | ≥ 800 | > 6,5 | Saint-Gobain (RimaxCeramicBeads) | |
| Microbilles en verre | | 500 | > 3,76 | - | |
| Microbilles en acier | | 700 | > 7,7 | - | |

Généralement, les microbilles 6 convenant pour l'invention ne sont pas en verre ou exclusivement en verre.

En particulier, les microbilles 6 représentent, en volume, par rapport au volume total de la chambre stationnaire 2 de 50% à 85%, de préférence de 55% à 70%.

Au sens de l'invention, « un volume de 50 à 85 % » comprend les valeurs suivantes : 50 ; 55 ; 60 ; 65 ; 70 ; 75 ; 80 ; 85 ; etc., ou tous intervalles compris entre ces valeurs.

La réaction de synthèse de solkétal est réalisée à chaud, c'est à dire à une température supérieure ou égale à 50°C, de préférence supérieure ou égale à 52°C et idéalement supérieure ou égale à 56°C afin d'obtenir un meilleur rendement.

En effet, la température d'ébullition de l'acétone est de 56,05°C à pression atmosphérique. C'est pourquoi, de manière avantageuse, la température de réaction au sein du broyeur est aux alentours de 56°C.

Selon un premier mode de réalisation, afin d'obtenir cette température d'au moins 50°C et de préférence 56°C, les réactifs de départ et/ou la composition de départ sont préalablement chauffés. Ainsi, il n'est pas nécessaire de chauffer ou d'avoir un dispositif de chauffage au sein ou autour du broyeur tridimensionnel à microbilles.

Par exemple, selon ce mode de réalisation, lors de l'étape préalable (0), la composition de départ est chauffée à une température supérieure ou égale à 50°C, de préférence supérieure ou égale à 56°C, de sorte que la température lors de l'étape de broyage (1) soit supérieure ou égale à 50°C, de préférence supérieure ou égale à 56°C.

En effet, dans la mesure où le temps de séjour des réactifs de départ ou de la composition de départ au sein du broyeur est très court (en général, inférieur ou égale à 5 minutes, de préférence inférieur ou égale à 2 minutes), le chauffage des réactifs de départ et/ou de la composition de départ est suffisant.

Ce mode de réalisation exige cependant une attention particulière notamment au moment du démarrage de la réaction lorsque le broyeur est froid ou au moment d'un changement de débit.

Selon un deuxième mode de réalisation, qui peut se combiner au premier, la température d'au moins 50°C et idéalement de 56°C peut être atteinte dans le broyeur tridimensionnel.

Pour cela, lors de l'étape de broyage (1), les réactifs de départ et/ou la composition de départ sont chauffés au sein du broyeur tridimensionnel à microbilles qui comprend au moins un dispositif de chauffage, de préférence au moins un dispositif de chauffage par induction 20. Ce mode de réalisation présente l'avantage d'une température de réaction plus précise, quel que soit le débit ou la température des réactifs de départ/de la composition de départ à l'entrée du broyeur (meilleur chauffage du flux formant le mélange de départ). Ce mode de réalisation est aussi particulièrement adapté à un mode de réalisation où plusieurs broyages successifs sont effectués sur le même mélange.

Par exemple et tel que cela est représenté sur la Fig.1, le ou les dispositifs de chauffage par induction 20 sont intégrés à l'intérieur de la chambre de broyage stationnaire 1 et permettent de chauffer au moins une zone de ladite chambre de broyage stationnaire 1.

Selon une caractéristique de l'invention, le ou les dispositifs de chauffage par induction 20 sont implantés à l'entrée de la chambre 1, c'est-à-dire aux alentours de la première extrémité 2 de sorte à pouvoir chauffer le flux de mélange initial (réactifs de départ/composition de départ) dès son introduction et permettre et/ou d'activer par conséquent la synthèse chimique du solkétal.

Selon un mode préféré de réalisation de l'invention, le dispositif de chauffage par induction 20 est porté par au moins une partie dudit agitateur 10, permettant la mise en mouvement rotatif du dispositif de chauffage par induction 20 autour de l'axe longitudinal XX.

Généralement, le dispositif de chauffage par induction 20 comprend :
- au moins un inducteur 21, apte à générer un champ magnétique, et
- au moins un suscepteur 22, conducteur d'électricité, qui est couplé audit inducteur 21 et qui est apte à être chauffé par celui-ci 21.

En particulier, l'inducteur 21 est une bobine ou un solénoïde présentant des spires qui entourent une partie de ladite tige 11 de l'agitateur 10, avantageusement un tronçon amont situé du côté de la première extrémité 2 comme cela est représenté sur la figure 1.

L'inducteur 21 est notamment apte à générer un champ magnétique ce qui va permettre le chauffage des matériaux conducteurs de son environnement, et en particulier le suscepteur 22 auquel il est couplé. En effet, le suscepteur 22, qui est conducteur d'électricité, est apte à capter le champ magnétique émis par l'inducteur.

L'ensemble bobine et suscepteur peuvent être mis en rotation par la tige 11.

Les autres organes de mélange 26 qui sont différents du premier organe de mélange 22, à savoir ils ne sont pas forcément conducteurs d'électricité, peuvent notamment être réalisés en fonte au chrome ou céramique de type oxyde de zirconium.

En se référant à la figure 1, ce premier organe de mélange 22 comporte en général une base solidarisée avec la tige 11 de l'agitateur 10. De préférence, l'inducteur 21 est implanté au niveau de cette base.

Généralement, le dispositif de chauffage par induction 20 est relié à un générateur de courant électrique alternatif disposé en dehors de ladite chambre de broyage 1 par l'intermédiaire d'au moins un moyen d'amenée de courant 27 qui est coaxiale à la tige 11 de l'agitateur 10.

En particulier, le générateur peut présenter une puissance allant de 5 à 15KW et de préférence de 10 kW avec une fréquence variant par exemple de 17 à 200 kHz. Il comporte un boîtier de capacité qui peut être en parallèle ou en série. A titre d'exemple, un générateur en série ID Partner référence IX3600 modèle PO8010 convient pour réaliser le broyeur selon l'invention.

En général, la chambre de broyage stationnaire 1 intègre un écran magnétique 23 disposé entre ledit inducteur 21 et ladite tige 11 de l'agitateur 10, de sorte à orienter le chauffage vers le mélange initial.

En effet, il se peut que l'agitateur 10 ou sa tige 11 soit fabriqué en matériau conducteur d'électricité et ainsi, afin d'éviter toute surchauffe de l'agitateur 10, il est préférable de protéger l'agitateur 10 ou au moins la partie tige 11 qui est entourée par l'inducteur 21.

Cet écran magnétique 23 présente également l'avantage de diriger le champ magnétique émis par la bobine 21 au premier organe de mélange 22 afin que toute la puissance soit concentrée à l'extérieur de l'inducteur et en particulier ne soit pas dirigée vers la tige 11. Ainsi la zone de chauffe est restreinte à la périphérie extérieure de la tige 11 et particulièrement concentrée sur le premier organe de mélange 22.

Un tel broyeur intégrant un dispositif de chauffage est notamment décrit dans la demande FR 18 54592.

Tel que mentionné ci-dessus, il est possible de combiner le premier et second mode de réalisation Ainsi, selon un troisième mode de réalisation, la température d'au moins 50°C et idéalement de 56°C, est atteinte en combinant un préchauffage préalable selon le premier mode de réalisation et un chauffage interne au broyeur selon le second mode de réalisation.

A titre d'exemple, le broyeur tridimensionnel à microbilles en phase liquide convenant pour réaliser le procédé selon l'invention peut correspondre à des broyeurs commercialisés par les sociétés WAB, gamme Dyno-Mill : Multi Lab, ECM et KD, NETZCH ou encore Alpine Hosokawa, par exemple, Agitated Media Mill AHM ou à ces types de broyeurs dans lequel un dispositif de chauffage tel que décrit ci-dessus a été intégré.

Le procédé de fabrication selon l'invention va désormais être décrit plus explicitement ci-dessous.

En particulier, ci-après, nous allons décrire plus en détail le mode de réalisation comprenant l'étape préalable (0) de préparation de la composition de départ, bien que celle-ci ne soit pas limitative de l'invention. En effet, comme déjà susmentionné, les réactifs de départ peuvent être introduits directement dans le broyeur 100.

Ainsi, de façon avantageuse, la fabrication du solkétal selon l'invention peut comprendre (0) une étape préalable de préparation de la composition de départ. En effet, il est généralement plus aisé d'un point de vue pratique de préparer la composition de départ comprenant les différents réactifs de départ dans les proportions voulues. Eventuellement, le catalyseur selon l'invention peut être ajouté ultérieurement dans le broyeur tridimensionnel 100.

Ainsi, le glycérol est mélangé avec de l'acétone selon un ratio molaire (glycérol : acétone) inférieur ou égale à 0,8, de préférence inférieur ou égale à 0,7.

Selon l'invention, un ratio molaire (glycérol : acétone) inférieur ou égale à 0,8, comprend les ratios suivants et tous intervalles compris entre ces valeurs : 0,8 ; 0,7 ; 0,6 ; 0,5 ; 0,4 ; 0,3 ; 0,2 ; 0,1, etc.

En particulier, le ratio molaire est également supérieur ou égale à 0,1, de préférence supérieur ou égale à 0,2 et typiquement supérieur ou égale à 0,3, comme par exemple de l'ordre de 0,5.

Typiquement, le ratio molaire (glycérol : acétone) est de l'ordre de 0,5. Contrairement aux procédés de l'art antérieur, il n'est pas nécessaire d'avoir un large excès d'acétone afin de réaliser la synthèse du solkétal selon l'invention.

La composition de départ est classiquement préparée par mélange des réactifs de départ, à savoir au moins l'acétone et le glycérol et de préférence le catalyseur dans un dispositif approprié, tel qu'un récipient ou une cuve, muni d'un système d'agitation (tel qu'agitateur magnétique, des pales d'agitation, etc.). Le dispositif ainsi que le système d'agitation pourront être adaptés par l'homme du métier en fonction de la quantité de solkétal à fabriquer.

Tel qu'indiqué ci-dessus, le glycérol et l'acétone sont mélangés afin de réaliser la réaction suivante :

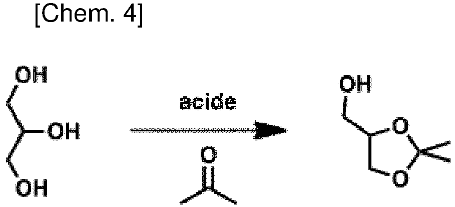

De façon connue, la viscosité du glycérol décroit très rapidement avec la température, mais également avec la teneur en eau. C'est pourquoi, de préférence, le glycérol servant pour le procédé de l'invention est anhydre ou présente une teneur en eau, en masse, par rapport à la masse totale du glycérol allant de 0 à 10%, de préférence allant de 0 à 5%.

Selon l'invention, du glycérol comprenant une quantité en eau allant de 0 à 10% inclut les valeurs suivantes ou tous intervalles entre ces valeurs : 0 ; 1 ; 2 ; 3 ; 4 ; 5 ; 6 ; 7 ; 8 ; 9 ou encore 10%.

La viscosité du glycérol pourra, selon des techniques connues de l'homme du métier, telles qu'à l'aide d'un viscosimètre à lame vibrante, être mesurée avant de réaliser le procédé selon l'invention et en particulier l'étape de broyage (1).

Le glycérol convenant pour la présente invention se trouve sous forme de liquide. Le glycérol de numéro CAS : 56-81-5 et commercialisé par exemple par la société Mon-droguiste.com de pureté supérieure ou égale à 99,5% convient pour réaliser le procédé de l'invention.

L'acétone convenant pour la présente invention se trouve sous forme de liquide. L'acétone de numéro CAS : 67-64-1 et commercialisée par exemple par la société Mon-droguiste.com de pureté supérieure ou égale à 99,5% convient pour réaliser le procédé de l'invention.

De préférence, l'acétone et le glycérol présentent une pureté élevée, en général supérieure ou égale à 90%, en particulier supérieure ou égale à 95% et typiquement supérieure ou égale à 99%, voire supérieure ou égale à 99,9%.

Le catalyseur convenant pour le procédé selon l'invention est choisi parmi un acide de Lewis dur (accepteur de doublet) comprenant au moins un métal de transition. En particulier, le métal de transition est choisi parmi le fer, l'aluminium, le manganèse ou le chrome. A titre d'exemple, le catalyseur selon l'invention peut être choisi parmi : FeCl₃ comme FeCl₃.6H₂O, AlCl₃, CrCl₃, MnSO₄ ou un de leurs mélanges.

Le catalyseur représente, en masse, par rapport à la masse totale de la composition de départ composée de l'acétone, du glycérol et du catalyseur, de 0,02% à 1%, de préférence de 0,03% à 0,08% et typiquement de 0,03% à 0,05%, tel que 0,044%.

Une fois que la composition de départ est préparée, celle-ci est amenée au broyeur tridimensionnel à microbilles 100 par l'intermédiaire généralement de la pompe péristaltique à débit réglable via l'entrée 4. La pompe péristaltique permet de continuer le mélange de la composition de départ avant l'entrée dans la chambre stationnaire 1. En outre, tel qu'indiqué précédemment, cette pompe permet d'introduire la composition de départ dans la chambre 1 avec un débit de passage contrôlé.

Généralement, la composition de départ est introduite à un débit de passage de 5 à 130 L/h, de préférence de 10 à 100 L/h et typiquement de 10 à 90 L/h.

Selon l'invention, un « débit de passage allant de 5 à 130 L/h » comprend les valeurs suivantes et tous intervalles entre ces valeurs : 5 ; 6 ; 7 ; 8 ; 9 ; 10 ; 11 ; 12 ; 13 ; 14 ; 15 ; 16 ; 17 ; 18 ; 19 ; 20 ; 21 ; 22 ; 23 ; 24 ; 25 ; 26 ; 27 ; 28 ; 29 ; 30 ; 35 ; 40 ;45 ; 50 ; 55 ; 60 ; 65 ; 70 ; 75 ; 80 ; 85 ; 90 ; 95 ; 100 ; 105 ; 110 ; 115 ; 120 ; 125 ; 130.

Une fois que la composition de départ est introduite dans la chambre 1, l'étape de broyage (1) débute.

Sous l'effet du courant créé par le débit de passage, la composition de départ parcourt la chambre stationnaire 1 de l'entrée 4 à la sortie 5, tout en étant mise en mouvement par l'agitateur 10 qui permet un brassage intense de cette composition avec les microbilles et, le cas échéant, avec les disques 22 ; 26, les doigts 28, etc., le long de la paroi interne de la chambre 1.

La vitesse de rotation de l'agitateur pourra par exemple varier de 10 à 150 Pi rad/s, de préférence de 40 à 100 et en particulier de 60 à 70 Pi rad/s et est en particulier d'au moins 60 Pi rad/s comme 63 Pi rad/s.

Selon l'invention, une vitesse de rotation allant de 10 à 150 Pi rad/s comprend les valeurs suivantes et tous intervalles entre ces valeurs : 10 ; 20 ; 30 ; 40 ; 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 ; 110 ; 120 ; 130 ; 140 ; 150.Le temps de séjour de la suspension de départ est inférieur ou égal à 15 min.

Selon l'invention, un temps de séjour inférieur ou égal à 15 minutes comprend les valeurs suivantes et tous intervalles entre ces valeurs : 15 min ; 10 min ; 11 min ; 10 min ; 9 min ; 8 min ; 7 ; min ; 6 min ; 5 min ; 4 min ; 3 min ; 2 min ; 1 min ; 55 sec ; 50 sec ; 45 sec ; 40 sec ; 35 sec ; 30 sec ; 25 sec ; 20 sec ; 15 sec ; 10 sec ; 5 sec, etc.

De préférence, le temps de séjour dans le broyeur est inférieur ou égal à 10 minutes, et va notamment de 30 secondes à 8 minutes et en particulier de 50 secondes à 5 minutes.

Il est en effet inhérent au volume apparent des billes et au débit de passage. Par exemple, si le volume total apparent des billes est de 270 cm³ (billes de masse volumique apparente de 3,7 g/cm³) et que le débit d'introduction de la suspension est de 45 L/h, soit 12,45 cm³/s, alors le temps de séjour de la suspension dans la chambre 1 est estimé à environ 22 secondes. Par conséquent, le temps de séjour peut être avantageusement réglé, par exemple en contrôlant la masse volumique apparente des microbilles, ainsi que le débit de passage.

On entend par « volume apparent » le volume des microbilles incluant l'air interstitiel entre les billes. La masse volumique apparente est le rapport entre la masse des microbilles et le volume apparent.

De préférence, le volume apparent des microbilles va de 250 mL à 450 mL, de préférence de 300 mL à 400 mL et est typiquement de 330 mL à 360 mL. Ce volume apparent des microbilles convient par exemple pour un broyeur tridimensionnel comprenant une chambre stationnaire 1 de 500 mL.

En outre, en jouant sur la taille des microbilles et le débit de passage, la synthèse du solkétal peut être améliorée.

De préférence, la pression lors de l'étape de broyage (1) va de 0,05 à 20 MPa, de préférence de 0,08 à 0,5 MPa et est typiquement de l'ordre de 0,1 MPa.

L'étape de broyage peut être réalisée en mode continu ou en mode discontinu en un ou plusieurs passages (mode pendulaire ou en recirculation). Lorsqu'elle est réalisée en mode discontinu, le nombre de passages de la composition de départ peut être de 1 à 10, préférentiellement de 1 à 5 (à savoir, après un premier passage, on récupère la composition finale à la sortie 6 et on la réintroduit, grâce à la pompe, dans la chambre 1 via l'entrée 4 pour permettre un deuxième passage). En particulier, le nombre de passages de la suspension de départ est de 1.

En effet, les inventeurs ont remarqué qu'un seul et unique passage dans le broyeur à microbilles, malgré un temps de séjour très court, permettait d'obtenir en sortie 5 une composition finale comprenant majoritairement du solkétal.

Ainsi, cette étape de broyage est réalisée de préférence en mode continu.

Avantageusement, cette étape de broyage se déroule à une température supérieure ou égale à 50°C, à savoir le plus souvent à une température supérieure ou égale à 56°C qui est la température de synthèse du solkétal et ce afin d'obtenir un meilleur rendement.

Une fois l'étape de broyage réalisée, (1) la composition finale est récupérée à la sortie 5 du broyeur 100. Cette composition finale peut comprendre des traces des réactifs de départ qui n'ont pas réagi, tels que par exemple le catalyseur ou encore un sous-produit, le 1,3-O-isoprpylidene-glycerol si la température de réaction ou les concentrations des réactifs de départ n'étaient pas dans les gammes idéales de réaction.

Le procédé selon l'invention permet d'obtenir un rendement en solkétal supérieur ou égal à 80%, de préférence supérieur ou égale à 85% et en particulier supérieur ou égale à 90%.

En général, le procédé selon l'invention permet d'obtenir un rendement en solkétal supérieur ou égal à 99% (conditions expérimentales optimisées).

De façon connue, le solkétal pourra être séparé du milieu réactionnel (des réactions de départ et/ou de la composition de départ, voire du ou des autres sous-produits formés) par des méthodes bien connues de l'homme du métier. Ces méthodes peuvent être par exemple par extraction, par évaporation du solvant (acétone) ou encore par distillation.

Le solkétal pourra par ailleurs être purifié si nécessaire par des techniques là aussi bien connues de l'homme du métier, comme par distillation ou par chromatographie sur colonne sur gel de silice ou encore par chromatographie liquide haute performance (HPLC).

### EXEMPLES

La description des essais ci-dessous est donnée à titre d'exemple purement illustratif et non limitatif.

### Matières premières

Pour les essais, les matières premières de départ sont :
Glycérol (Fluorochem, Hadfield, UK) Lot FCB019493
FeCl₃.6H₂O (Fluorochem, Hadfield, UK) Lot FCB048596
Acétone>99% (Sigma-Aldrich, Steinheim, Allemagne), Lot #STBJ2303

### Broyeur selon l'invention

Les essais ont été mis en œuvre dans un broyeur tridimensionnel à microbilles Dynomill ECM AP-05 de la Société Willy A. Bachofen AG (WAB), qui contient 1,235kg de microbilles, et qui a été adapté de sorte à inclure un dispositif de chauffage 20 selon l'invention tel que représenté sur la figure 1. A savoir, le broyeur comprend un dispositif de chauffage positionné à l'entrée de la chambre stationnaire, et le premier organe de mélange agit en tant que suscepteur (brevet FR18 54592).

En particulier, le dispositif de chauffage présente les caractéristiques suivantes :

**[Tableau 2]**

| **Eléments** | **Caractéristiques** |
|---|---|
| Générateur | Générateur de puissance 10kW avec une fréquence variant de 17 à 200 kHz/générateur en série IDPartner référence IX3600 modèle PO8010. |
| Inducteur | Fils de Litz multibrins et résinés pour être démontables. Câble de chez IDPartner 300 brins Litz Cu 9,425 mm² 6x50x0,2mm. |
| Suscepteur | Organe de mélange tel que décrit dans le document US 5 597 126 (fig.4) en acier inoxydable Phyterm^{®} 260 équivalent Inox ferrique Kara de chez ArcelorMittal nuance K44. |
| Ecran magnétique | Tore cylindrique en Fluxtrol^{®} |
| Moyen d'amenée de courant | La tige 11 a été modifiée pour intégrer l'amenée de courant coaxiale 3mm² en cuivre. Ce câble coaxial modifie le centre de gravité de la tige ; il est ainsi équilibré en le compensant par l'insertion de vis en tungstène. |
| Thermocouples | De type K à l'entrée et en sortie de la chambre de broyage. |
| Contacteur | Contacteur rotatif en cuivre. |

Les microbilles sont en oxyde de zirconium et présentent un diamètre de 0,5 mm. Les caractéristiques des microbilles utilisées pour les essais sont résumées dans le tableau 3 ci-dessous :

**[Tableau 3]**

| | **Billes** | 0.45/0.55 mm |
|---|---|---|
| Composition (% en masse) | | 93% ZrO₂ |
| | | 5% Y₂O₃ |
| | | 2% autres |
| Masse volumique spécifique | | 6.0 g/cc |
| Masse volumique apparente | | 3.7 kg/l |
| Dureté Vickers | | 1250 HV1 |

Les microbilles de 0,5mm sont notamment commercialisées sous le nom de marque Zirmil^{®} Y Ceramic Beadspar la société Saint-Gobain.

La chambre de broyage du broyeur présente une capacité de 514 mL et est remplie, en volume, par rapport à son volume total et en fonction des essais, de 167 ou 334 mL des microbilles décrites ci-dessus.

En fonctionnement, les microbilles sont mises en agitation par un agitateur à une vitesse de rotation pouvant varier selon les exemples de 6 ou 8 m/s. L'agitateur comporte en outre des disques mélangeurs en fonte au chrome.

### Procédure générale de mise en œuvre selon l'invention (3^{ème} mode de réalisation)

### Préparation de la composition de départ avec préchauffage :

Un mélange de glycérol (425,0 g, 4,62 mol), de catalyseur et d'acétone (5,54 mol ; 6,93mol ; 9,24 mol ou 13,86 mol) sont agités magnétiquement et vigoureusement à 25°C, 40°C ou 56 °C dans un ballon tricols à fond arrondi d'1 litre équipé d'un condenseur (les conditions expérimentales sont illustrées sur le tableau 4). En particulier, la composition de départ de l'exemple 10 du tableau 4 est préchauffée à 40°C et celle de l'exemple 14, à 25°C, tandis que les compositions des autres exemples sont préchauffées à 56°C.

### Introduction au sein du broyeur tridimensionnel :

Le milieu réactionnel est pompé à l'aide d'une pompe péristaltique à un débit de 14 L/h ou de 42L/h et le flux est introduit dans le broyeur tridimensionnel Dynomill ECM AP-05 décrit ci-dessus préalablement chauffé ou non selon les exemples réalisés. En particulier, le dispositif de chauffage 20 n'est pas actionné pour l'exemple 14, ou permet de chauffer à une température de 40°C pour l'exemple 10 et à une température de 56°C pour les autres exemples du tableau 4.

Après un certain temps de séjour dans le broyeur, le milieu réactionnel est analysé comme décrit ci-après.

### Analyse des échantillons

Les analyses des molécules issues de la réaction ont été effectuée par chromatographie en phase gazeuse avec un chromatographe de marque Hewlett Packard (14009 Arcade, New York, United States). La chaîne se compose d'un système d'injection manuel équipé d'un septum (SPI), d'une colonne Supelco 2-8047-U colonne capillaire (15m x0.25mm i.d. and 0.25 µm épaisseurs de film, Alltech Part No.31163-01), d'un four, d'un détecteur à ionisation de flamme (FID, 70 eV, 300 µA, and 250 °C) et d'un système d'acquisition.

### Préparation de l'échantillon

Pour chaque échantillon, le milieu réactionnel (50 µL) et une solution n-décane - acétone (0.009 M dans acétone) sont mélangés.

### Calibration

L'étalonnage s'effectue en injectant plusieurs solutions contenant le solkétal à une concentration variable (3.65×10⁻⁴ M - 4.5625×10⁻⁵ M) et la solution n-décane (0.009 M dans acétone) - acétone à une concentration fixe.

Le solkétal (50 µL) et une solution n-décane - acétone (1:1, v/v) sont mélangés et représente une concentration de solkétal de 3.65×10⁻⁴ M et une concentration de n-décane de 0.009 M. Une fraction de ce mélange (50 µL) est diluée par une solution de n-décane - acétone (50 µL, 1:1, v/v) et représente une concentration de solkétal de 1.825×10⁻⁴ M et une concentration de n-décane de 0.009 M. Une fraction de ce mélange (50 µL) est diluée par une solution de n-décane - acétone (50 µL, 1:1, v/v) et représente une concentration de solkétal de 9.125×10⁻⁵ M et une concentration de n-décane de 0.009 M. Une fraction de ce mélange (50 µL) est diluée par une solution de n-décane - acétone (50 µL, 1:1, v/v) et représente une concentration de solkétal de 4.5625×10⁻⁵ M et une concentration de n-décane de 0.009 M.

### Analyse des échantillons

Le gaz vecteur utilisé est l'hydrogène à un débit de 1 mL.min⁻¹. Les échantillons (2 µL) sont injectés manuellement avec injecteur équipé d'un septum (SPI). La température de l'injecteur est réglée à 250°C avec une température de four de 70 °C pendant 1 min et une montée en température de 20 °C.min⁻¹ jusqu'à une température de 250 °C pendant 10 min.

L'analyse qualitative des composés a été réalisée par comparaison des temps de rétention avec des standards purs (acétone, n-décane, solkétal, glycérol). Le temps de rétention de l'acétone est de 0,417 min et les temps de rétention du n-décane, du solkétal et du glycérol sont respectivement de 1,33 min, 2,36 min et 3,61 min. L'analyse qualitative des composés a été réalisée sur la base de la courbe d'étalonnage.

### Conditions réactionnelle utilisées et résultats

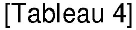

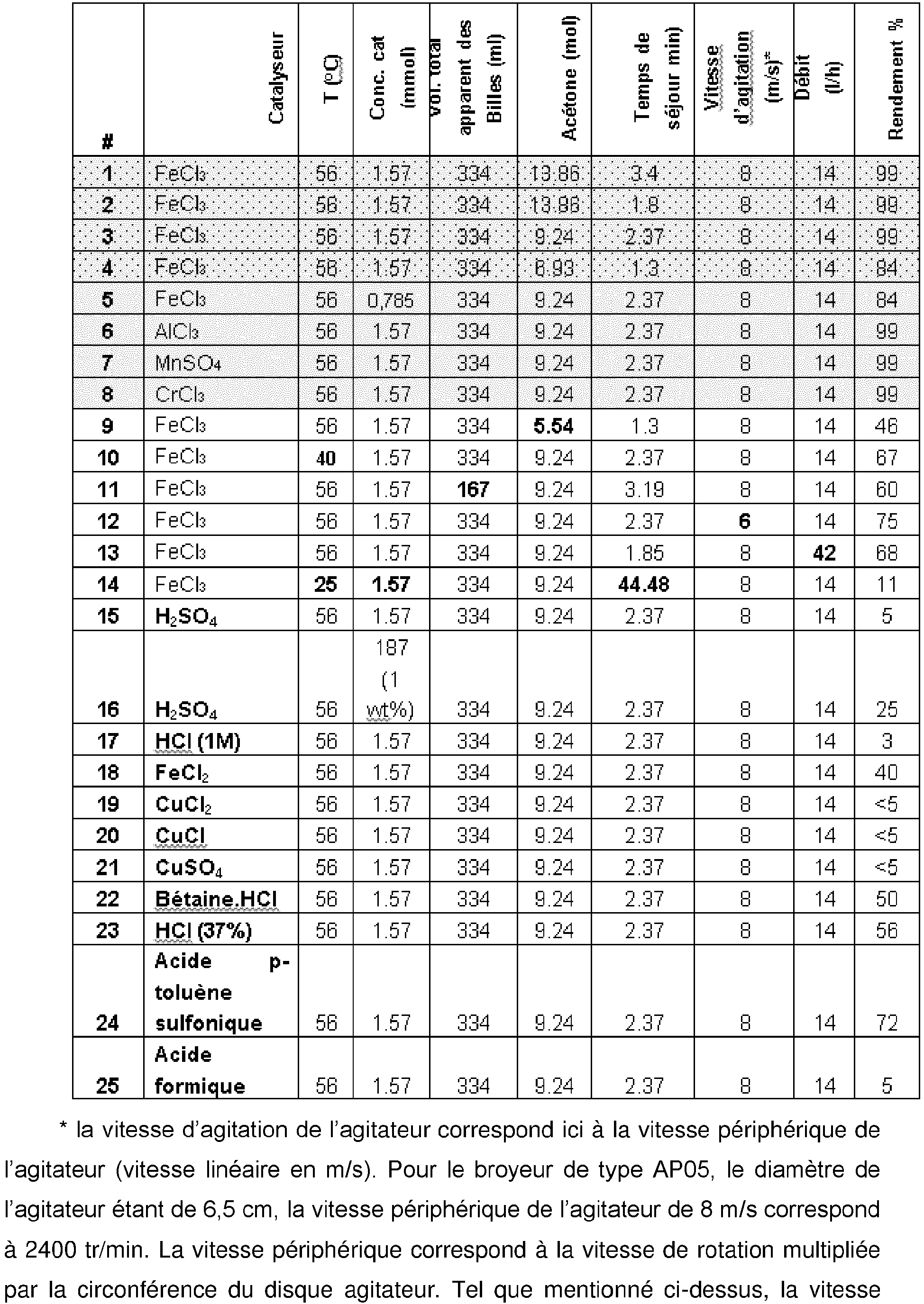

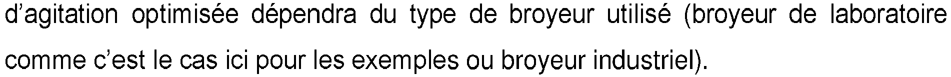

Comme on peut le constater, le procédé selon l'invention permet d'obtenir de très haut rendement en fonction notamment des conditions expérimentales (choix du catalyseur non arbitraire, température avantageusement de 56°C, volume des microbilles, concentration en acétone, etc.).

L'exemple 13 montre par exemple qu'un temps de séjour inférieur à 2 min (1,85 min) avec un débit de 42 L/h ne permet pas d'avoir un rendement en solkétal de 99% (mais de 68%), alors que l'exemple 2 montre qu'un temps de séjour de 1,8 min avec un débit de 14 L/h et une quantité plus importante en acétone (13,86 mol contre 9,24 mol pour l'exemple 11) permet d'obtenir un rendement de 99%.

D'une manière générale, il a été constaté par les inventeurs que plus le temps de séjour est long au sein de la chambre stationnaire du broyeur, meilleur est le rendement.

Il apparaît également que le choix du catalyseur est important et qu'un catalyseur acide dur de Lewis comprenant au moins un métal de transition comme FeCl₃, AlCl₃, CrCl₃, MnSO₄ ou un de leurs mélanges permet d'obtenir de très bons rendements, contrairement aux autres catalyseurs testés.

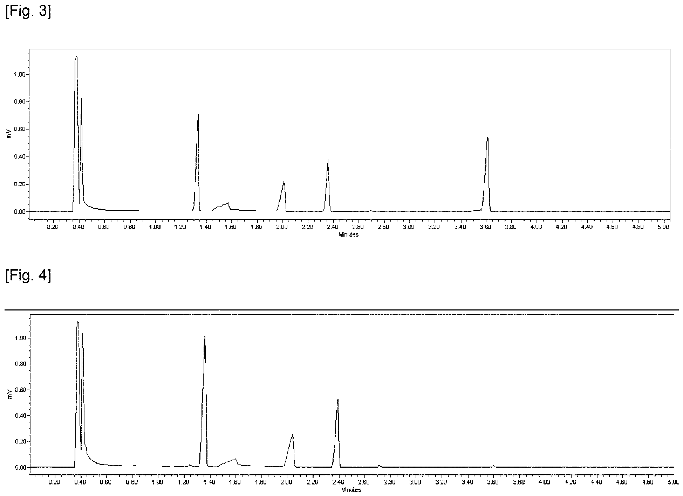

Également, la Fig.3 et la Fig.4 correspondant à l'exemple 3 montre qu'au bout d'un temps de séjour de 1 min (Fig.3), le procédé selon l'invention permet la synthèse de solkétal (temps de rétention de 2,356 min) mais qu'il reste également dans la composition finale du glycérol (temps de rétention de 3,607 min) ; tandis qu'avec un temps de séjour de 2,37 minutes (Fig.4), on n'observe plus le pic correspondant au glycérol. Ainsi, la réaction est totale et a permis d'obtenir du solkétal.

Bien évidemment, les paramètres du procédé (concentration des réactifs de départ, débit de passage, etc.) peuvent être optimisés afin d'obtenir une synthèse du solkétal en continue, à savoir en un seul passage. Par exemple, l'emploi d'un débit de passage plus faible permet d'augmenter par exemple le temps de séjour des réactifs de départ/composition de départ dans la chambre de broyage et ainsi permet d'augmenter le rendement en solkétal en un seul passage.

## Revendications

1. Procédé de fabrication du solkétal ((2,2-Diméthyl-1,3-dioxolan-4-yl)méthanol) qui comprend au moins les étapes suivantes :
(1) le broyage des réactifs suivants, dits réactifs de départ comprenant au moins : du glycérol, un catalyseur choisi parmi un acide de Lewis dur comprenant au moins un métal de transition, et de l'acétone, le ratio molaire (glycérol) : (acétone) étant inférieur ou égal à 0,8, à une température ambiante supérieure ou égale à 50°C, dans un broyeur tridimensionnel à microbilles en phase liquide pendant un temps de séjour inférieur ou égal à 15 minutes;
(2) la récupération en sortie du broyeur d'une composition finale comprenant du solkétal et le cas échéant, un ou des sous-produits correspondant au(x) réactif(s) de départ n'ayant pas réagi(s) et/ou au 1,3-O-isopropylidene-glycérol, et
(3) optionnellement, la séparation du solkétal du ou desdits sous-produits.

2. Procédé de fabrication selon la revendication 1, dans lequel la température lors de l'étape de broyage (1) est supérieure ou égale à 56°C.

3. Procédé de fabrication selon la revendication 1 ou 2, dans lequel le temps de séjour lors de l'étape de broyage (1) est inférieur ou égal à 10 minutes, de préférence inférieur ou égal à 5 minutes.

4. Procédé de fabrication selon l'une quelconque des revendications précédentes, comprenant une étape préalable (0) dans laquelle les réactifs de départ dont au moins le glycérol et l'acétone et de préférence le catalyseur acide de Lewis sont préalablement mélangés pour former une composition de départ.

5. Procédé de fabrication selon la revendication 4, dans lequel lors de l'étape préalable (0), la composition de départ est préalablement chauffée à une température supérieure ou égale à 50°C, de préférence supérieure ou égale à 56°C, de sorte que la température lors de l'étape de broyage (1) soit supérieure ou égale à 50°C, de préférence supérieure ou égale à 56°C.

6. Procédé de fabrication selon l'une quelconque des revendications précédentes, dans lequel lors de l'étape de broyage (1), les réactifs de départ sont chauffés au sein du broyeur tridimensionnel à microbilles qui comprend un dispositif de chauffage, de préférence un dispositif de chauffage par induction.

7. Procédé de fabrication selon l'une quelconque des revendications précédentes, dans lequel la pression lors de l'étape de broyage (1) va de 0,05 à 20MPa, de préférence de 0,08 à 0,5 MPa et est typiquement de l'ordre de 0,1 MPa.

8. Procédé de fabrication selon l'une quelconque des revendications précédentes, dans lequel le catalyseur acide de Lewis dur comprenant au moins un métal de transition est choisi parmi : FeCl₃, AlCl₃, CrCl₃, MnSO₄ ou un de leurs mélanges.

9. Procédé de fabrication selon l'une quelconque des revendications précédentes, dans lequel le glycérol est anhydre ou présente une teneur en eau, en masse, par rapport à la masse totale du glycérol allant de 0 à 10%, de préférence de 0 à 5%.

10. Procédé de fabrication selon l'une quelconque des revendications précédentes, dans lequel les microbilles sont de forme sphérique et présentent un diamètre moyen allant de 0,05 mm à 4 mm, de préférence de 0,2 à 3 mm, en particulier de 0,3 à 2 mm et typiquement de l'ordre de 0,5 à 1 mm.

11. Procédé de fabrication selon l'une quelconque des revendications précédentes, dans lequel les microbilles présentent une dureté de Vickers mesurée selon la norme EN ISO 6507-1 supérieure ou égale à 900 HV1, de préférence allant de 900 HV1 à 1600 HV1, typiquement allant de 1000 à 1400 HV1.

12. Procédé de fabrication selon l'une quelconque des revendications précédentes, dans lequel les microbilles présentent une masse volumique réelle allant de 2 à 15 g/cm³.

13. Procédé de fabrication selon l'une quelconque des revendications précédentes, dans lequel le broyeur tridimensionnel à microbilles comprend au moins :
- une chambre de broyage stationnaire de forme générale cylindrique s'étendant selon un axe longitudinal XX, ladite chambre étant remplie au moins, en partie, par lesdites microbilles et comprend : à une première extrémité au moins une entrée servant à introduire lesdits réactifs de départ, et à une seconde extrémité, une sortie comportant un moyen de séparation apte à n'évacuer que ladite composition finale formée dans ladite chambre ; et
- un agitateur, disposé dans la chambre de broyage stationnaire, se présentant sous forme d'une tige allongée selon l'axe longitudinal XX, ledit agitateur étant apte à mettre en mouvement l'ensemble microbilles/réactifs de départ.

14. Procédé de fabrication selon la revendication 13, dans lequel les microbilles représentent, en volume, par rapport au volume total de la chambre stationnaire de 50% à 85%, de préférence de 55% à 70%.

15. Procédé de fabrication selon l'une quelconque des revendications précédentes, dans lequel le broyeur fonctionne en continu.

## Patentansprüche

1. Verfahren zur Herstellung von Solketal ((2,2-Dimethyl-1,3-dioxolan-4-yl)methanol), das mindestens die folgenden Schritte umfasst:
(1) Mahlen der folgenden Reagenzien, der so genannten Ausgangsreagenzien, die mindestens die Folgenden umfassen: Glycerin, einen aus einer mindestens ein Übergangsmetall umfassenden harten Lewissäure ausgewählten Katalysator und Aceton, wobei das Molverhältnis (Glycerin) : (Aceton) kleiner oder gleich 0,8 ist, bei einer Umgebungstemperatur von mehr als oder gleich 50 °C in einer Planeten-Mikrokugelmühle in flüssiger Phase während einer Verweildauer von weniger als oder gleich 15 Minuten;
(2) Gewinnung einer Endzusammensetzung, die Solketal und gegebenenfalls ein oder mehrere Nebenprodukte, die dem nicht umgesetzten Ausgangsreagenz bzw. den nicht umgesetzten Ausgangsreagenzien und/oder 1,3-0-Isopropylidenglycerin entsprechen, umfasst, am Ausgang der Mühle und
(3) gegebenenfalls Abtrennung des Solketals von dem bzw. den Nebenprodukt(en).

2. Herstellungsverfahren nach Anspruch 1, wobei die Temperatur während des Mahlschritts (1) höher als oder gleich 56 °C ist.

3. Herstellungsverfahren nach Anspruch 1 oder 2, wobei die Verweildauer während des Mahlschritts (1) weniger als oder gleich 10 Minuten, vorzugsweise weniger als oder gleich 5 Minuten, beträgt.

4. Herstellungsverfahren nach einem der vorhergehenden Ansprüche, das einen vorherigen Schritt (0) umfasst, in dem die Ausgangsreagenzien, darunter mindestens Glycerin und Aceton und vorzugsweise der Lewissäure-Katalysator, zuvor unter Bildung einer Ausgangszusammensetzung gemischt werden.

5. Herstellungsverfahren nach Anspruch 4, wobei die Ausgangszusammensetzung während des vorherigen Schritts (0) zuvor auf eine Temperatur von mehr als oder gleich 50 °C, vorzugsweise mehr als oder gleich 56 °C, erwärmt wird, so dass die Temperatur während des Mahlschritts (1) höher oder gleich 50 °C, vorzugsweise höher oder gleich 56 °C, ist.

6. Herstellungsverfahren nach einem der vorhergehenden Ansprüche, wobei die Ausgangsreagenzien während des Mahlschritts (1) im Inneren der Planeten-Mikrokugelmühle, die eine Heizvorrichtung, vorzugsweise eine Induktionsheizvorrichtung, umfasst, erwärmt werden.

7. Herstellungsverfahren nach einem der vorhergehenden Ansprüche, wobei der Druck während des Mahlschritts (1) von 0,05 bis 20 MPa, vorzugsweise von 0,08 bis 0,5 MPa reicht und üblicherweise in der Größenordnung von 0,1 MPa liegt.

8. Herstellungsverfahren nach einem der vorhergehenden Ansprüche, wobei der mindestens ein Übergangsmetall umfassende harte Lewissäure-Katalysator ausgewählt ist aus: FeCl₃, AlCl₃, CrCl₃, MnSO₄ oder einem ihrer Gemische.

9. Herstellungsverfahren nach einem der vorhergehenden Ansprüche, wobei das Glycerin wasserfrei ist oder einen Massengehalt an Wasser, bezogen auf die Gesamtmasse des Glycerins, im Bereich von 0 bis 10 %, vorzugsweise von 0 bis 5 %, aufweist.

10. Herstellungsverfahren nach einem der vorhergehenden Ansprüche, wobei die Mikrokugeln sphärisch geformt sind und einen mittleren Durchmesser im Bereich von 0,05 mm bis 4 mm, vorzugsweise von 0,2 bis 3 mm, insbesondere von 0,3 bis 2 mm und üblicherweise in der Größenordnung von 0,5 bis 1 mm aufweisen.

11. Herstellungsverfahren nach einem der vorhergehenden Ansprüche, wobei die Mikrokugeln eine gemäß der Norm EN ISO 6507-1 gemessene Vickers-Härte von mehr als oder gleich 900 HV1, vorzugsweise im Bereich von 900 HV1 bis 1600 HV1, üblicherweise im Bereich von 1000 bis 1400 HV1, aufweisen.

12. Herstellungsverfahren nach einem der vorhergehenden Ansprüche, wobei die Mikrokugeln eine tatsächliche Dichte im Bereich von 2 bis 15 g/cm³ aufweisen.

13. Herstellungsverfahren nach einem der vorhergehenden Ansprüche, wobei die Planeten-Mikrokugelmühle mindestens Folgendes umfasst:
- einen stationären Mahlraum mit im Großen und Ganzen zylindrischer Form, der sich entlang einer Längsachse XX erstreckt, wobei der Raum zumindest zum Teil mit den Mikrokugeln gefüllt ist und Folgendes umfasst: an einem ersten Ende mindestens einen Einlass, der dazu dient, die Ausgangsreagenzien einzubringen, und an einem zweiten Ende einen Auslass, der ein Abtrennmittel umfasst, das dazu geeignet ist, nur die im Raum gebildete Endzusammensetzung abzulassen; und
- ein im stationären Mahlraum angeordnetes Rührwerk in Form eines entlang der Längsachse XX langgestreckten Stabs, wobei das Rührwerk dazu geeignet ist, die Gesamtheit aus Mikrokugeln/Ausgangsreagenzien in Bewegung zu setzen.

14. Herstellungsverfahren nach Anspruch 13, wobei die Mikrokugeln, bezogen auf das Gesamtvolumen des stationären Raums, 50 bis 85 Volumen-%, vorzugsweise 55 bis 70 Volumen-%, ausmachen.

15. Herstellungsverfahren nach einem der vorhergehenden Ansprüche, wobei die Mühle kontinuierlich arbeitet.p

## Claims

1. Method for preparing solketal ((2,2-dimethyl-1,3-dioxolan-4-yl)methanol) comprising at least one of the following steps:
(1) milling the following reactants, referred to as initial reactants, comprising at least: glycerol, a catalyst selected from a hard Lewis acid containing at least one transition metal, and acetone, the glycerol:acetone molar ratio being less than or equal to 0.8, at an ambient temperature greater than or equal to 50°C, in a three-dimensional mill with microbeads in liquid phase for a residence time of less than or equal to 15 minutes,
(2) collecting, at the outlet of the mill, an final composition comprising the solketal and, where applicable, one or more byproducts corresponding to unreacted initial reactant(s) and/or 1,3-0-isopropylideneglycerol, and
(3) optionally, separating the solketal from said by-product(s).

2. Method of preparation according to Claim 1, wherein the temperature during the milling step (1) is greater than or equal to 56°C.

3. Method of preparation according to Claim 1 or 2, wherein the residence time during the milling step (1) is less than or equal to 10 minutes, preferably less than or equal to 5 minutes.

4. Method of preparation according to any one of the preceding claims, comprising a preliminary step (0) wherein the initial reactants, including at least glycerol and acetone and preferably the Lewis acid catalyst, are premixed to form an initial composition.

5. Method of preparation according to Claim 4, wherein, during the preliminary step (0), the initial composition is preheated to a temperature greater than or equal to 50°C, preferably greater than or equal to 56°C, so that the temperature during the milling step (1) is greater than or equal to 50°C, preferably greater than or equal to 56°C.

6. Method of preparation according to any one of the preceding claims, wherein, during the milling step (1), the initial reactants are heated inside the three-dimensional mill with microbeads, which includes a heating device, preferably an induction heating device.

7. Method of preparation according to any one of the preceding claims, wherein the pressure during the milling step (1) is within a range from 0.05 to 20 MPa, preferably from 0.08 to 0.5 MPa, and is typically of the order of 0.1 MPa.

8. Method of preparation according to any one of the preceding claims, wherein the hard Lewis acid catalyst containing at least one transition metal is chosen from: FeCl₃, AlCl₃, CrCl₃, MnSO₄ or a mixture thereof.

9. Method of preparation according to any one of the preceding claims, wherein the glycerol is anhydrous or has a water content by mass, relative to the total mass of the glycerol, within a range from 0 to 10%, preferably from 0 to 5%.

10. Method of preparation according to any one of the preceding claims, wherein the microbeads are spherical in shape and have an average diameter within a range from 0.05 mm to 4 mm, preferably from 0.2 to 3 mm, in particular from 0.3 to 2 mm and typically of the order of 0.5 to 1 mm.

11. Method of preparation according to any one of the preceding claims, wherein the microbeads have a Vickers hardness, measured in accordance with standard EN ISO 6507-1, greater than or equal to 900 HV1, preferably within a range from 900 HV1 to 1600 HV1, typically within a range from 1000 to 1400 HV1.

12. Method of preparation according to any one of the preceding claims, wherein the microbeads have real volumic mass within a range from 2 to 15 g/cm³,

13. Method of preparation according to any one of the preceding claims, wherein the three-dimensional mill with microbeads comprises at least:
- a stationary mill chamber generally cylindrical in shape, extending along a longitudinal axis XX, said chamber being filled, at least in part, with said microbeads and comprising: at one end at least one inlet used to introduce said initial reactants and, at the other end, an outlet comprising a separation means capable of discharging only said final composition formed in said chamber; and
- an agitator arranged in the stationary mill chamber and taking the form of a rod extending along the longitudinal axis XX, said agitator being capable of setting in motion the totality of the microbeads/initial reactants.

14. Method of preparation according to Claim 13, wherein the microbeads constitute by volume 50% to 85%, preferably 55% to 70%, of the total volume of the stationary chamber.

15. Method of preparation according to any one of the preceding claims, wherein the mill operates continuously.
